# EUROPEAN PATENT APPLICATION

(11) **EP 4 122 430 A1**
(43) Date of publication of application: **25.01.2023**
(21) Application number: 22175527.5
(22) Date of filing: 25.05.2022
(51) Int. Cl.: A61F 5/01

(54) **DEVICE FOR THE TREATMENT OF FOOT CONDITIONS**

(30) Priority: 22.07.2021 GB 202110563
(71) Applicant: Fernandes, Daniel Ferreira, 2855-620 Verdizela (PT)
(72) Inventor: Fernandes, Daniel Ferreira, 2855-620 Verdizela (PT)
(74) Representative: Meissner Bolte UK

(57) **Abstract**

There is described herein a device (1) for the treatment of foot conditions. The device comprising an anchor point (3) configured to be attached to an ankle of a user, a sole element (7) with a proximal end extending from the anchor point, and in use is configured to extend under the sole of a foot of the user, a toe raising element (9) extending from a distal end of the sole element, the toe raising element configured in use to pivot the toes above the toes resting position, wherein the toe raising element comprises a strap (11) configured to attach to the anchor point.

## Description

### Field of Invention

The present application concerns a device for the treatment of foot conditions, in particular for the treatment plantar fasciitis and Achilles tendonitis (the device may also be used to treat other foot conditions). The device is to be worn by a user during periods of inactivity such as sleep, or whilst at a desk to improve the condition of the foot.

### Background

Foot conditions can hamper an individual. These conditions include plantar fasciitis and Achilles tendonitis.

Plantar fasciitis is the inflammation of the fibrous tissue (the plantar fascia) along the bottom of the foot that connects the heel bone to the toes. Plantar fasciitis commonly causes sharp pain when the foot is used for weight bearing at the beginning of the day, or after long periods of inactivity. Typically, this pain reduces through use of the foot throughout the day. Plantar fasciitis is common with runners, and therefore this is one risk group. Additional risk factors include those who are overweight, or people who use shoes with inadequate support.

The Achilles is the largest tendon in the human body and connects the calf muscles to the heel bone. Achilles tendonitis is an inflammation or irritation of the tendon and can lead to weakening of the tendon. Achilles tendonitis may be caused by a sudden increase in the level of exercise, for example in the amount of running, particularly hill or fell running, squash playing, or basketball playing. It may also be common with older people as the Achilles weakens with age.

Devices for the treatment of these conditions have been developed previously, but require improvement. For example, socks for each of these conditions are currently used. It is common to use a knee high length sock with the toe of the sock connected to the top of the sock to raise the position of the toes. However, these devices have several drawbacks. Firstly, using a knee high sock means that as the knee flexes the tension to the toes changes, affecting the position of the toes. As such devices are often worn at night this can create problems. A further issue is that the connection between the toe of the sock and the top of the sock often does not raise each of the toes (or at least the four principal toes as in some individuals the little toe may have limited movement) equally. Indeed, these devices often provide significantly more pivoting of the big toe than any other toe. This means that they are not very effective.

Moreover, having the knee length sock often means that during the night the top of the sock slips down, and this also may affect the tension and thus the pivoting of the toes. For all of these reasons use of such devices provide inconsistent effects to the user.

The present invention seeks to provide a device that mitigates the above problems and so provides relief to user's suffering from such foot conditions. It is noted that this device is principally aimed at plantar fasciitis, but has been found to also benefit patients with Achilles tendonitis.

### Summary of Invention

According to one aspect there is provided a device for the treatment of foot conditions, the device comprising an anchor point configured to be attached to an ankle of a user, a sole element with a proximal end extending from the anchor point, and in use is configured to extend under the sole of a foot of the user, a toe raising element extending from a distal end of the sole element, the toe raising element configured in use to pivot the toes above the toes resting position, wherein the toe raising element comprises a strap configured to attach to the anchor point. The use of an anchor point configured to be attached to an ankle of a user is particularly advantageous. Typically, ankles include the narrowest point on the leg, and therefore by attaching the device to the ankle, and in particular to the narrowest point of the leg, the anchor point is made more secure - and reduces the risk of movement of the anchor point over time (for example during sleep). This therefore means that tension in the strap of the toe raising element is likely to be more uniform during use, and so the results of use may be more reliable.

Optionally, the toe raising element is configured to extend past the end of the user's toes. This allows the toe raising element to double back over the user's toes and act as a sling for the toes so as to pivot them upwards during use.

Optionally, in use the toe raising element is configured to fold back over the top of the user's toes when the strap of the toe raising element is attached to the anchor point during use. This allows the toes to be pivoted upwards, and therefore to lengthen the plantar fascia during use. The lengthening of the plantar fascia has been found to aid with treatment of plantar fasciitis.

Optionally, the width of the sole element at the distal end, at a position level with the user's toes, is at least commensurate with the width of the user's toes. This may allow each of the toes (in some user's excluding the little toe) to be pivoted.

Optionally, the proximal end of the toe raising element is configured to be less flexible than the sole element. This is particularly advantageous as having the distal end of the sole element to be less flexible has been found to provide tension on the toes more equally (possibly excluding the little toe depending on the user). More flexible distal ends have been found to transfer most tension onto just the big toe - and so result in substantially more pivoting of the big toe. Having a more rigid distal end addresses this problem by spreading the force more equally and so results in improved treatment. It is noted that the first aspect when combined with this optional feature may be advantageous even if the anchor point is no longer configured to anchor to the ankle, but is instead placed elsewhere.

Optionally, the toe raising element comprises an attachment point. This allows the strap to attach to the toe raising element.

Optionally, in use the strap of the toe raising element is attached to the anchor point, and the strap is doubled back and attached to the attachment point of the distal end of the toe raising element. This has been found to be easy to use, and an effective way of attaching the strap to ensure the tension is transferred to the toes.

Optionally, further comprising a fastening mechanism attached to the sole element, wherein in use the fastening mechanism is configured to wrap around the user's foot, to minimise movement of the device relative to the user's foot during use. This allows the device to function more reliably as it lessens the movement of the device relative to the foot.

Optionally, the fastening mechanism comprises two wings attached to either side of the sole element, wherein the two wings are configured to be attachable to one another so as to wrap around the user's foot during use. This has been found to be easy to use and to securely attach the device to the foot.

Optionally, the fastening mechanism comprises a single strap attached to a first side of the sole element, and configured to wrap around the user's foot and fasten to a second side of the sole element. This is an alternative way of attaching the device to the foot.

Optionally, the anchor point comprises a loop of material configured to be worn around the ankle of the user. This enables the device to be fastened more securely to the ankle.

Optionally, the inside of the loop comprises an adhesive strip, optionally wherein the adhesive strip comprises a silicone strip. The adhesive strip has been found to decrease movement of the device relative to the ankle, and therefore keep the force applied to the toes more consistent.

Optionally, the anchor point has an adjustable girth, such that the radius of the loop may be increased or decreased. This may allow a range of users of different sizes to use the device.

Optionally, the toe raising element pivots at least the position of the four largest toes of the user, optionally wherein all of the toes of the user are pivoted by the toe raising element.

Optionally, the device is configured for the treatment of plantar fasciitis and/or Achilles tendonitis. It is noted that the device may be used to treat other foot conditions.

Optionally, the anchor point, sole element, toe raising element and fastening mechanism comprise one or more fabric materials. This may allow the force to be transferred to the toes of the user, whilst minimising any user discomfort.

Optionally, the sole element and the toe raising element are of unitary construction. This may reduce wear and tear, and may be more efficient at transferring tension to the toes. This may also be more comfortable for the user as non-unitary construction may lead to small amounts of friction on the user's foot at the position of the join.

Optionally, a posterior surface of the sole element comprises a grasping element configured to hold an object. The grasping element may be particularly advantageous as it may hold objects below a user's foot (such as ice or a ball for treatment.

Optionally, the grasping element comprises a loop.

Optionally, the grasping element is configured to hold an ice pack below the sole element such that the user's foot is cooled during use.

According to a second aspect there is provided a method of treating a patient with plantar fasciitis and/or Achilles tendonitis, the method comprising the user attaching the anchor point of the device of the first aspect to their ankle, and attaching the toe raising element to the anchor point so as to pivot the position of the user's toes. This may be particularly advantageous as treating foot conditions.

Optionally, further comprising attaching the toe raising element to the anchor point at a position such that the sole element and/or the toe raising element are taught.

Optionally, the device is worn overnight.

### Brief Description of Figures

Figure 1 shows a plan view of the device according to a first embodiment.
Figure 2 shows a plant view of the device according to a second embodiment.
Figure 3 shows a perspective view of the device of the first embodiment in use around a user's foot.
Figure 4 shows the perspective view of Figure 3 of the device of the first embodiment, however the user's foot has been removed from figure 4.
Figure 5 shows a side view of the device of the first embodiment in use around a user's foot.

### Description of Figures

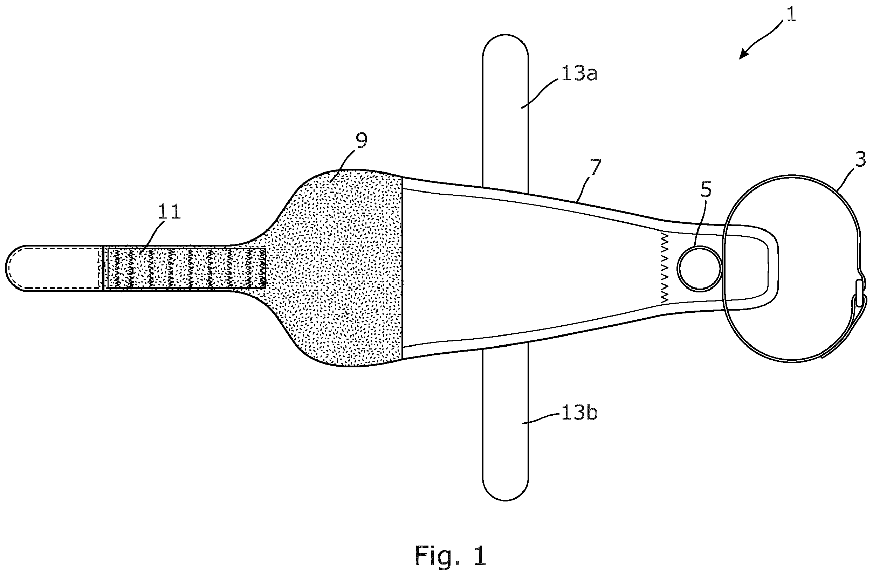

Figure 1 shows a plan view of the device according to a first embodiment. Figure 1 shows a device 1 for the treatment of foot conditions, the device comprising an anchor point 3 configured to be attached to an ankle of a user, a sole element 7 with a proximal end extending from the anchor point, and in use is configured to extend under the sole of a foot of the user, a toe raising element 9 extending from a distal end of the sole element, the toe raising element configured in use to pivot the toes above the toes resting position, wherein the toe raising element comprises a strap 11 configured to attach to the anchor point. These elements are those required, whereas the other features portrayed in Figure 1 are optional.

The device of Figure 1 comprises an anchor point 3, an attachment point 5, a sole element 7, a toe raising element 9, a strap 11, and a fastening mechanism 13, comprising a first part 13a, and a second part 13b.

The anchor point 3 comprises a loop of material and is configured to be worn around the ankle of the user. The anchor point 3 comprises a strap with a buckle to allow the anchor point 3 to be detachable form the user. The buckle may also allow the diameter of the anchor point 3 to be adjustable. The buckle may allow the material of anchor point 3 to loop back on itself. This may allow use of an adhesive such as Velcro or the like to keep the anchor point in place. Alternatively, the strap of the anchor point may have within it a number of holes and a belt-buckle style attachment may be used. As the ankle is often the narrowest portion of a human lower leg (the calf is wider than it, as is the protuberance forming part of the lower ankle) configuring the anchor point to attach to the ankle is greatly advantageous as the anchor point is substantially less likely to move along the leg due to it being situated at the narrowest part. This is a significant improvement on anchor points located elsewhere (such as the knee) where they are substantially less stable.

The sole element 7 is attached to the anchor point 3. This attachment is the proximal end of the sole element. The sole element is configured in use to extend from the anchor point 3 and down to the bottom of the heel, and then along the base of the foot. As such the sole element 7 is shaped to approximately correspond to the footprint of a user's foot. The proximal end of the sole element 7 is therefore narrower than the distal end of the sole element as feet typically widen from the heel towards the toes. The sole element 7 is comprised of a fabric material, and may be relatively flexible to allow it to be used by users with a variety of different foot sizes. The dashed lines on sole element 7 in Figure 1 indicate approximately where the heel of the user should sit during use. This may aid the user in correctly positioning the device upon their foot.

Figure 1 also shows fastening mechanism 13 comprising a first part 13a and a second part 13b. The first and second parts may be referred to as wings. The wings extend from a central portion of the sole element 7. The wings of the fastening mechanism extend perpendicularly to a central longitudinal axis of the sole element (the longitudinal axis being along the centre of the sole element in the direction of proximal end to distal end of the sole element 7). The wings are configured to attach to one another. In Figure 1 the wings 13a and 13b are shown as being attachable via an adhesive such as Velcro or the like. Alternatively, a buckle may be used. The wings are configured to be adjustable such that feet of different depths may be contained. The fastening mechanism is configured to the strap users foot to the device such that movement of the device relative to the foot is decreased.

The toe raising element 9 is attached to the distal end of the sole element 7. In particular, the proximal end of the toe raising element 9 is attached to the sole element 7. The toe raising element is hatched to show that it is comprised of less flexible (or more rigid) material than the sole element. The toe raising element is configured extend past the user's toes and to fold over the user's toes to pivot the toes upwards from the toes' natural resting position. The more rigid material allows all of the toes (possibly excluding the little toe in some users - as this toe is often less flexible) to be pivoted. If more flexible material was used it has been found that this often pivots only the big toe - and is therefore substantially less beneficial. The reduced flexibility (or increased rigidity) of the toe raising element (or at least the proximal portion thereof) is therefore greatly beneficial. The toe raising element 9 also comprises a strap 11. The strap 11 is at the distal end of the toe raising element.

In use the strap 11 is configured to attach to the anchor point. In the embodiment shown in Figure 1 the strap is configured to pass through attachment point 5, adjacent and attached to the anchor point 3. The strap is then configured to pass back and attach either to itself or to another part of the toe raising element. The position at which the strap attaches may be referred to as comprising an attachment means. In use it is advantageous for the strap to be taught such that the toe raising element then pivots the toes of the user upwards of their natural resting position.

It is noted that in Figure 1 both the sole element and the toe raising element are of unitary construction. The exact point at which the sole element ends and the toe raising element begins may be altered depending on the foot condition being treated, and the user's specific physiology. It is beneficial for these elements to be of unitary construction to reduce friction on the foot of the user at the join, in order to increase comfort.

It is also noted that the features of Figure 1 are not inextricably linked together. For example, the toe raising element being more rigid, or less flexible, than the sole element is not tied to use of the fastening mechanism. Each of the features of Figure 1 are separable, and offer improvements and advantages in their own right. However, it is also noted that these features in combination combine to offer the particularly advantageous embodiment shown in Figure 1 as the features work together to offer more than the sum of their parts.

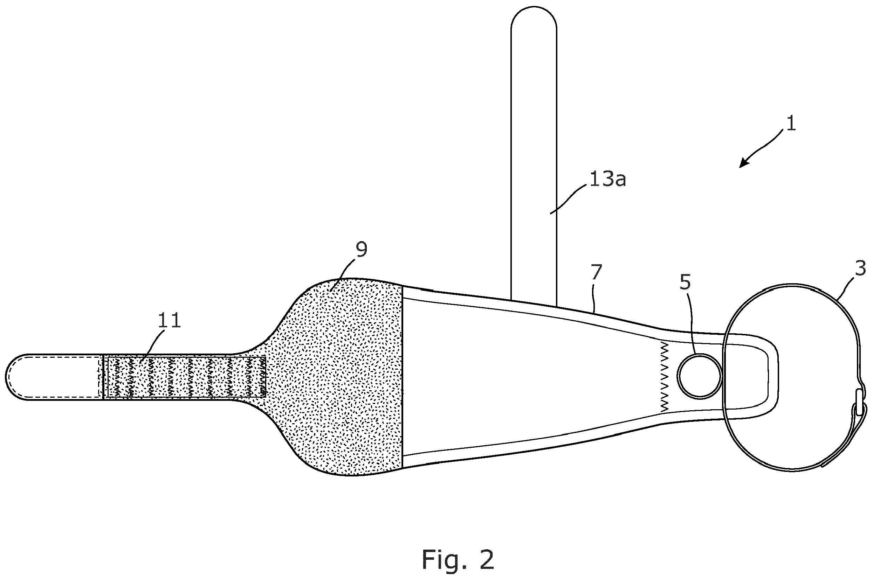

Figure 2 shows a second embodiment of the device in a plan view. The device of Figure 2 is identical to the device of Figure 1 except that the fastening mechanism has been changed. Figure 2 shows just a single wing attached to one side of the sole element 7. The single wing is shown as being perpendicular the longitudinal axis of the sole element 7. The single wing is configured to wrap around the user's foot and attach to the opposing side of the sole element 7. Figure 2 shows a buckle used to attach the sole wing 13 to the sole element 7 however alternative attachment means may be used.

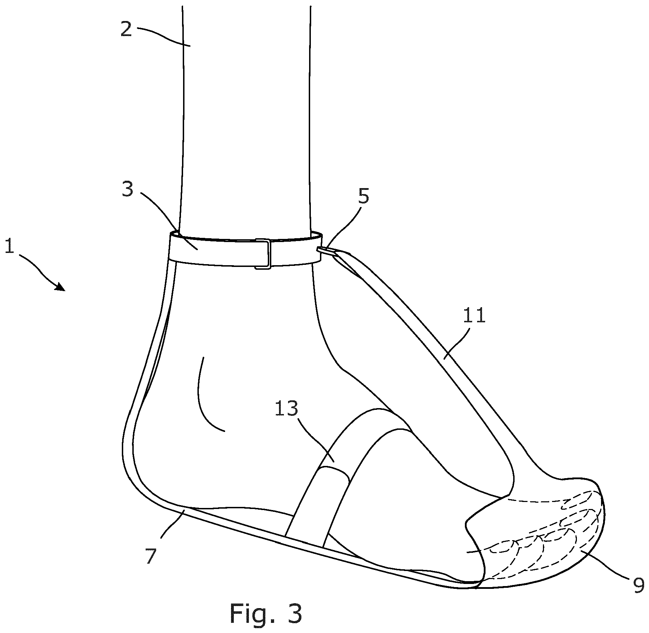

Figure 3 shows a perspective view of the device in use on a user's foot. The anchor point 3 is shown looped around the user's ankle. As the ankle is the narrowest point of the leg the anchor point is substantially stable in situ. The sole element 7 is shown extending from the anchor point 3 along the heel of the user and then under the sole of the user's foot. The fastening mechanism 13 extending from the central portion of the sole element 7 wraps around the user's foot such that movement of the device relative to the foot is minimised. The toe raising element 9 is shown extending past the end of the user's toes. The toe raising element 9 is shown doubling back over the user's toes to act as a sling to pivot the user's toes above the toes' normal resting position. The toes are shown as being raised in Figure 3. The toe raising element is held in place by strap 11. The strap 11 extends to the attachment point 5 on the anchor point 3. The strap may attach to the anchor point 3 in any suitable way. Figure 3 shows the strap 11 looping through attachment point 5 and passing back towards the toe raising element 9. The strap 11 may attach to itself or any other part of the toe raising element to secure the strap 11 in place. The strap 11 in Figure 3 is shown as being taught such that it pulls the toe raising element 9 up so that the toe raising element 9 can in turn pivot the toes upward.

Figure 3 shows that all of the toes of the user's foot are raised by the device 1. The toe raising element (in some embodiments just the proximal end of the toe raising element) is less flexible than other elements of the device 1. For example, it may be less flexible than the element 7, or alternatively the strap 11. This increased rigidity (or lower flexibility) allows all of the toes to be raised by the toe raising element. Prior systems with greater flexibility in the toe raising element allow for only the big toe to be raised - which is considered a problem as it does not treat the foot condition as efficiently.

Figure 3 shows the fastening mechanism of Figure 1, however alternative fastening mechanisms such as the one shown in Figure 2 may alternatively be used.

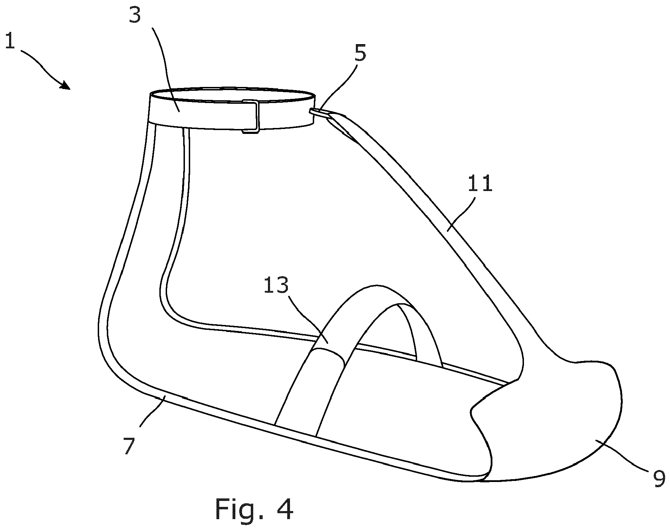

Figure 4 shows the device 1 in it's in-use form. However, the foot of the user has been removed from the view of Figure 4. Figure 4 shows that the sole element is flexible enough to allow for the bend adjacent the user's heel, as well as to give enough flexibility to allow for users of differing foot sizes to use the device effectively. Figure 4 also shows the arch formed by the fastening mechanism 13. This arch constrains the foot such that relative movement between the foot and device is minimised. It is noted that in this and all other embodiments the fastening mechanism 13 may be positioned at any position along the sole element 7. For example, the fastening mechanism may be positioned closer to the ankle of the user such that it is situated near to the bony protuberance of the ankle.

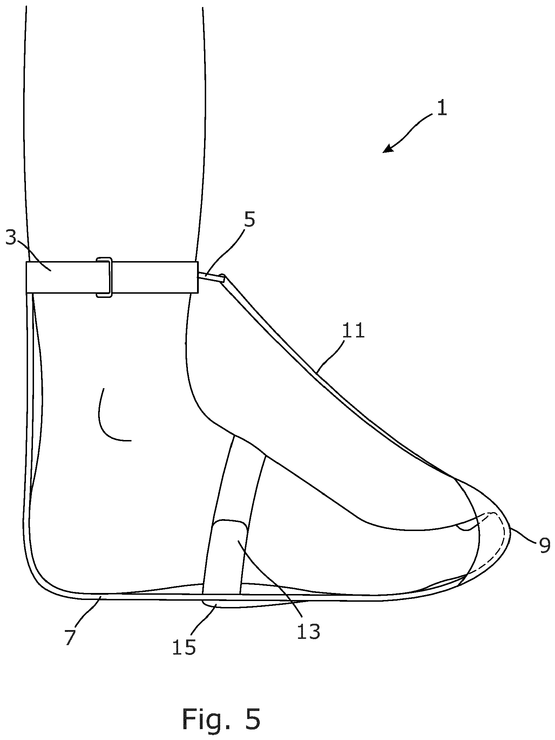

Figure 5 shows a side view of the view of Figure 3. Figure 5 shows the grasping element 15 positioned on the posterior surface of the sole element 7. The grasping element shown comprises a loop of material. This material may be elasticated. Alternatively, the grasping element 15 may form a pocket or any other suitable structure. The grasping element is configured to hold an object below the sole of a user's foot. For example, the grasping element may hold ice below the sole of the user's foot. This is particularly advantageous as ice reduces inflammation and therefore has been found to be helpful in combatting the symptoms of plantar fasciitis. Therefore, placing ice in the grasping element for a prescribed duration of time during use of the device for treatment of plantar fasciitis is particularly advantageous as it may reduce inflammation of the plantar fascia.

Also shown in Figure 5 is the strap 11 being fed through the attachment point 5 of the anchor point 3 and then going back on itself towards the distal end of the toe raising element 9. The strap attaches to either itself or to the toe raising element, for example through an adhesive, for example Velcro.

The device shown in the Figures as described above may be used for treating a patient with plantar fasciitis and/or Achilles tendonitis. The method of use may comprise the user attaching the anchor point of the device to their ankle, and attaching the toe raising element to the anchor point so as to pivot the position of the user's toes. This is advantageous as it allows the toes to be raised such that the symptoms of plantar fasciitis may not develop, and the root causes are addressed (e.g. tightness/inflammation) by stretching the plantar fascia for a duration of time. The method may comprise attaching the toe raising element to the anchor point at a position such that the sole element and/or the toe raising element are taught. This tightness may allow the force to be conveyed to the toes so that the pivoting of the toes is achieved effectively and consistently for the prescribed period. That period may include wearing the device overnight, or alternatively for significant periods of rest such as when working at a desk, or sitting on the sofa etc. As part of this method of use ice may be placed inside the grasping element under the sole of the foot. The ice may only be placed under the sole of the foot for a prescribed time on the basis of medical advice, for example by placing the ice under the sole of the foot for a period of approximately five minutes at the beginning of use of the device. This may numb the area and may allow the device to work even more efficiently in treatment of the foot condition.

It will be appreciated that the device shown and described herein may be adapted in a number of ways whilst still being within the scope of protection. Moreover, the Figures are purely illustrative of two specific embodiments of the device. Alternative devices include devices that do not comprise a fastening mechanism. Whilst the fastening mechanism is preferable it is not essential to the functioning of the device. Similarly, the attachment point on the anchor point may be replaced or removed. Another alternative embodiment may comprise an alternate form of toe raising element - such as a shorter toe raising element that pivots and supports the toes, but does not double back over the toes to form a sling, and instead offers support more akin to a hammock. These and other alternate embodiments are clear alternatives to those explicitly described herein, and would be entirely contemplated by the person skilled in the art when considering how to implement the invention set out in the claims on the basis of the teachings above. All such alternative embodiments are considered to be within the scope of the claims.

### Clauses

1. A device for the treatment of foot conditions, the device comprising:
   an anchor point configured to be attached to an ankle of a user;
   a sole element with a proximal end extending from the anchor point, and in use is configured to extend under the sole of a foot of the user;
   a toe raising element extending from a distal end of the sole element, the toe raising element configured in use to pivot the toes above the toes resting position;
   wherein the toe raising element comprises a strap configured to attach to the anchor point.
2. The device of clause 1, wherein the toe raising element is configured to extend past the end of the user's toes.
3. The device of clause 2, wherein in use the toe raising element is configured to fold back over the top of the user's toes when the strap of the toe raising element is attached to the anchor point during use.
4. The device of either of clauses 2 or 3, wherein the width of the sole element at the distal end, at a position level with the user's toes, is at least commensurate with the width of the user's toes.
5. The device of any preceding clause, wherein the proximal end of the toe raising element is configured to be less flexible than the sole element.
6. The device of clause 5, wherein the toe raising element comprises an attachment means.
7. The device of clause 6, when dependent on clause 3, wherein in use with the strap of the toe raising element attached to the anchor point, the strap is configured to doubled back and attach to the attachment means of toe raising element.
8. The device of any preceding clause, further comprising a fastening mechanism attached to the sole element, wherein in use the fastening mechanism is configured to wrap around the user's foot, to minimise movement of the device relative to the user's foot during use.
9. The device of clause 8, wherein the fastening mechanism comprises two wings attached to either side of the sole element, wherein the two wings are configured to be attachable to one another so as to wrap around the user's foot during use.
10. The device of clause 8, wherein the fastening mechanism comprises a single strap attached to a first side of the sole element, and configured to wrap around the user's foot and fasten to a second side of the sole element.
11. The device of any preceding clause, wherein the anchor point comprises a loop of material configured to be worn around the ankle of the user.
12. The device of clause 10, wherein the inside of the loop comprises an adhesive strip, optionally wherein the adhesive strip comprises a silicone strip.
13. The device of clause 11, wherein the anchor point has an adjustable girth, such that the radius of the loop may be increased or decreased.
14. The device of any preceding clause, wherein the toe raising element pivots at least the position of the four largest toes of the user, optionally wherein all of the toes of the user are pivoted by the toe raising element.
15. The device of any preceding clause, wherein the device is configured for the treatment of plantar fasciitis and/or Achilles tendonitis.
16. The device of any preceding clause, wherein the anchor point, sole element, toe raising element and fastening mechanism comprise one or more fabric materials.
17. The device of any preceding clause, wherein the sole element and the toe raising element are of unitary construction.
18. The device of any preceding clause, wherein a posterior surface of the sole element comprises a grasping element configured to hold an object.
19. The device of clause 18, wherein the grasping element comprises a loop.
20. The device of clause 18 or 19, wherein the grasping element is configured to hold an ice pack below the sole element such that the user's foot is cooled during use.
21. A method of treating a patient with plantar fasciitis and/or Achilles tendonitis, the method comprising the user attaching the anchor point of the device of any of clauses 1-14 to their ankle, and attaching the toe raising element to the anchor point so as to pivot the position of the user's toes.
22. The method of clause 22, the method comprising attaching the toe raising element to the anchor point at a position such that the sole element and/or the toe raising element are taught.
23. The method of either of clauses 21 or 22, wherein the device is worn overnight, and/or whilst the user is in a sedentary position.

## Claims

1. A device for the treatment of foot conditions, the device comprising:
an anchor point configured to be attached to an ankle of a user;
a sole element with a proximal end extending from the anchor point, and in use is configured to extend under the sole of a foot of the user;
a toe raising element extending from a distal end of the sole element, the toe raising element configured in use to pivot the toes above the toes resting position;
wherein the toe raising element comprises a strap configured to attach to the anchor point.

2. The device of claim 1, wherein the toe raising element is configured to extend past the end of the user's toes

3. The device of claim 2, wherein in use the toe raising element is configured to fold back over the top of the user's toes when the strap of the toe raising element is attached to the anchor point during use.

4. The device of either of claims 2 or 3, wherein the width of the sole element at the distal end, at a position level with the user's toes, is at least commensurate with the width of the user's toes.

5. The device of any preceding claim, wherein the proximal end of the toe raising element is configured to be less flexible than the sole element, optionally wherein the toe raising element comprises an attachment means, further optionally wherein in use with the strap of the toe raising element attached to the anchor point, the strap is configured to doubled back and attach to the attachment means of toe raising element.

6. The device of any preceding claim, further comprising a fastening mechanism attached to the sole element, wherein in use the fastening mechanism is configured to wrap around the user's foot, to minimise movement of the device relative to the user's foot during use.

7. The device of claim 6, wherein the fastening mechanism comprises two wings attached to either side of the sole element, wherein the two wings are configured to be attachable to one another so as to wrap around the user's foot during use; or alternatively
wherein the fastening mechanism comprises a single strap attached to a first side of the sole element, and configured to wrap around the user's foot and fasten to a second side of the sole element.

8. The device of any preceding claim, wherein the anchor point comprises a loop of material configured to be worn around the ankle of the user, optionally wherein the inside of the loop comprises an adhesive strip, optionally wherein the adhesive strip comprises a silicone strip, optionally wherein the anchor point has an adjustable girth, such that the radius of the loop may be increased or decreased.

9. The device of any preceding claim, wherein the toe raising element pivots at least the position of the four largest toes of the user, optionally wherein all of the toes of the user are pivoted by the toe raising element.

10. The device of any preceding claim, wherein the device is configured for the treatment of plantar fasciitis and/or Achilles tendonitis.

11. The device of any preceding claim, wherein the anchor point, sole element, toe raising element and fastening mechanism comprise one or more fabric materials.

12. The device of any preceding claim, wherein the sole element and the toe raising element are of unitary construction.

13. The device of any preceding claim, wherein a posterior surface of the sole element comprises a grasping element configured to hold an object

14. The device of claim 13, wherein the grasping element comprises a loop.

15. The device of claim 13 or 14, wherein the grasping element is configured to hold an ice pack below the sole element such that the user's foot is cooled during use.
